# EUROPEAN PATENT SPECIFICATION

(11) **EP 4 140 913 B1**
(45) Date of publication and mention of the grant of the patent: **18.06.2025**
(21) Application number: 21792407.5
(22) Date of filing: 08.04.2021
(51) Int. Cl.: B65D 47/42, A61K 8/02, A61K 9/08, A61M 35/00, A61Q 19/00, B65D 39/00, B65D 51/18, B65D 51/32

(54) **DRUG SOLUTION APPLICATOR CONTAINER PROVIDED WITH AN INSIDE PLUG**
MEDIKAMENTENLÖSUNGSAPPLIKATORBEHÄLTER MIT INNENSTOPFEN
RÉCIPIENT D'APPLICATION DE SOLUTION MÉDICAMENTEUSE ÉQUIPÉ D'UN BOUCHON INTÉRIEUR

(30) Priority: 20.04.2020 JP 2020074489; 06.07.2020 JP 2020116176; 14.12.2020 JP 2020206479
(43) Date of publication of application: 01.03.2023
(73) Proprietor: Taisei Kako Co., Ltd., Kita-ku Osaka-shi Osaka 531-0072 (JP)
(72) Inventor: OGAWA Yukihiro, Ibaraki-shi Osaka 567-0054 (JP); MIYATAKE Satoshi, Ibaraki-shi Osaka 567-0054 (JP); NOMA Shota, Ibaraki-shi Osaka 567-0054 (JP)
(74) Representative: HKW Intellectual Property PartG mbB
(86) International application number: PCT/JP2021/014860
(87) International publication number: WO 2021/215256

(56) References cited:
- EP-B1- 1 066 771
- AU-A1- 2012 279 752
- JP-A- 2006 001 047
- JP-A- 2008 023 877
- JP-A- H0 952 487
- JP-U- H 075 778
- JP-U- H0 184 778
- JP-U- S62 136 378
- US-A1- 2009 226 240

## Description

### BACKGROUND OF THE INVENTION

### FIELD OF THE INVENTION

The present invention relates to an inside stopper for a medical solution applying container used for a variety of medical solutions such as the one applied to human skin and also relates to a medical solution applying container including the same.

### BACKGROUND ART

There has been conventionally developed a type of medical solution applying container not only for storing a medical solution but also for applying the medical solution to human skin or so forth with a brush attached thereto (e.g., JP 2008 - 169 163 A).

AU 2012 279 752 A1 discloses an applicator comprising a solution container according to the preamble of claim 1 and which comprises an opening, and a columnar brush member formed by bundling synthetic fibers in a columnar shape. The columnar brush member is disposed at the opening of the solution container, a tip portion of the columnar brush member at an outside of the solution container has a fan shape expanding in a perpendicular lateral direction against a pillar axial lengthwise direction, and a thickness of the fan-shaped tip portion of the columnar brush member decreases in a perpendicular lengthwise direction against the pillar axial lengthwise direction toward the tip portion of the columnar brush member. The liquid applicator of the present invention has the fan-shaped tip portion so that irritation on an affected part may be decreased and a liquid tinea medicine may be applied to the affected part.

In general, such a medical solution applying container as described above includes a medical solution container body made of a material with plasticity and stores a medical solution therein. The medical solution container body is pinched or squeezed by a user when the medical solution is applied to the skin or so forth of the user. The amount of medical solution extracted from the medical solution applying container is configured to be adjusted in accordance with adjustment in level of pinching or squeezing by the user.

Specifically, when the medical solution container body is squeezed strongly, a large amount of medical solution is absorbed into the brush and is then extracted therefrom; contrarily, when the medical solution container body is squeezed lightly, a small amount of medical solution is absorbed into the brush and is then extracted therefrom.

However, such a conventional medical solution applying container has had a drawback of difficulty in adjusting the extraction amount of medical solution.

For example, a user with a relatively weak grip cannot pinch or squeeze the medical solution container body with an appropriate level of grip even if trying to adjust the extraction amount of medical solution. When the actual extraction amount of medical solution is lesser than expected, the user has to settle for the lesser amount; contrarily, when the actual extraction amount of medical solution is too much than expected, the medical solution is wasted by that much.

The present invention has been produced in view of the drawback described above. It is an object of the present invention to provide a medical solution applying container including the inside stopper.

### SUMMARY OF THE INVENTION

According to the present invention, a medical solution applying container is provided according to claim 1 An inside stopper is attached at a lower end portion thereof to a medical solution container body for storing a medical solution. The inside stopper is provided with a brush attached to an upper end portion thereof so as to apply the medical solution. The inside stopper includes a brush inserted-and-mounted hole and a pressing-for-maintaining portion. The brush inserted-and-mounted hole is a hole to which the brush is inserted and mounted. The pressing-for-maintaining portion is provided in a lower end portion of the brush inserted-and-mounted hole. The pressing-for-maintaining portion presses a lateral periphery of the brush so as to maintain a position of the brush with respect to the inside stopper. The pressing-for-maintaining portion includes a tongue piece, and a gap is formed at the tongue piece.

The pressing-for-maintaining portion includes a plurality of tongue pieces. Besides, the plurality of tongue pieces are disposed through a plurality of gaps formed therebetween. Each of the plurality of gaps extends along the brush inserted-and-mounted hole.

Preferably, a plurality of vertical ribs are formed to extend from a surface of the lower end portion of the brush inserted-and-mounted hole to inner surfaces of the plurality of tongue pieces.

Preferably, each of the plurality of tongue pieces is provided with a hook portion directed toward the upper end portion in a contact position with the lateral periphery of the brush.

Preferably, the brush inserted-and-mounted hole is provided with a receiving portion on an upper end thereof. The receiving portion is contacted by a contact portion protruding from a middle portion of the lateral periphery of the brush.

Preferably, the brush inserted-and-mounted hole is provided with an engaging portion in a middle portion thereof. The engaging portion is engaged with the middle portion of the lateral periphery of the brush.

Preferably, the brush inserted-and-mounted hole is provided with a recessed groove on an inner surface thereof. The recessed groove extends from an opening end of the brush inserted-and-mounted hole to a lower end thereof on which the pressing-for-maintaining portion is provided.

### ADVANTAGEOUS EFFECTS OF INVENTION

In the inside stopper for a medical solution applying container according to the present invention, the brush inserted-and-mounted hole, to which the brush is inserted and mounted, is provided at the lower end portion thereof with the pressing-for-maintaining portion that presses the lateral periphery of the brush so as to maintain the position of the brush with respect to the inside stopper. Accordingly, depending on to what extent the lower end portion of the brush is protruded and exposed from the brush inserted-and-mounted hole to the interior of the medical solution container body in which the medical solution is stored, it is possible to adjust the amount of the medical solution permeating the lower end portion of the brush.

Consequently, the extraction amount of the medical solution can be herein easily adjusted, unlike in a conventional medical solution applying container, without necessity of adjusting the extraction amount of the medical solution depending on the level of pinching or squeezing the medical solution container body.

### BRIEF DESCRIPTION OF THE DRAWINGS

Referring now to the attached drawings which form a part of this original disclosure:
FIG. 1 is a cross-sectional view of a medical solution applying container 10 according to an exemplary embodiment to which the present invention is applied;
FIG. 2 is a perspective view of a medical solution container body 12;
FIG. 3 is a perspective view of an inside stopper 14;
FIG. 4 is a cross-sectional view of the inside stopper 14;
FIG. 5 is a perspective view of the inside stopper 14 shown cut away in part;
FIG. 6 is a perspective view of a brush 16;
FIG. 7 is a perspective view of a cap 18;
FIG. 8 is a cross-sectional view of the inside stopper 14 according to modification 1;
FIG. 9 is a cross-sectional view of the inside stopper 14 shown cut away in part according to the modification 1;
FIG. 10 is a cross-sectional view of the inside stopper 14 according to modification 2;
FIG. 11 is a perspective view of the inside stopper 14 shown cut away in part according to the modification 2;
FIG. 12 is a perspective view of the brush 16 according to the modification 2;
FIG. 13 is a cross-sectional view of a combined condition between the inside stopper 14 and the brush 16 according to the modification 2;
FIG. 14 is a perspective view of the brush 16 according to modification 3;
FIG. 15 is a cross-sectional view of a combined condition between the inside stopper 14 and the brush 16 according to the modification 3;
FIG. 16 is a cross-sectional view of the inside stopper 14 according to modification 4;
FIG. 17 is a perspective view of the brush 16 according to the modification 4;
FIG. 18 is a cross-sectional view of a combined condition between the inside stopper 14 and the brush 16 according to the modification 4;
FIG. 19 is a plan view of the inside stopper 14 according to modification 5;
FIG. 20 is a cross-sectional view of the inside stopper 14 shown cut away in part according to the modification 5;
FIG. 21 is a cross-sectional view of a combined condition between the inside stopper 14 and the brush 16 according to the modification 5;
FIG. 22 is a top view of an alternative example of the inside stopper 14 according to the modification 5;
FIG. 23 is a perspective view of the alternative example of the inside stopper 14 shown cut away in part according to the modification 5;
FIG. 24 is a cross-sectional view of the inside stopper 14 according to modification 6;
FIG. 25 is a cross-sectional view of the inside stopper 14 shown cut away in part according to the modification 6;
FIG. 26 is a cross-sectional view of a combined condition between the inside stopper 14 and the brush 16 according to the modification 6;
FIG. 27 is a cross-sectional view of the inside stopper 14 according to modification 7; and
FIG. 28 is a perspective view of the inside stopper 14 according to the modification 7.

### DETAILED DESCRIPTION OF EMBODIMENTS

### (Configuration of Medical Solution Applying Container 10)

An exemplary configuration of the medical solution applying container 10 to which the present invention is applied will be hereinafter explained with reference to drawings.

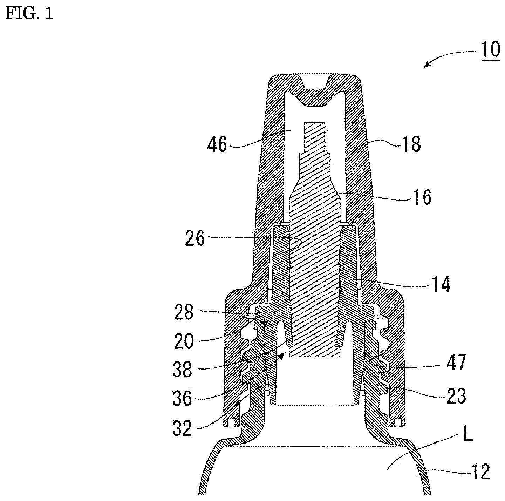

As shown in FIG. 1, the medical solution applying container 10 is mainly composed of the medical solution container body 12, the inside stopper 14, the brush 16, and the cap 18.

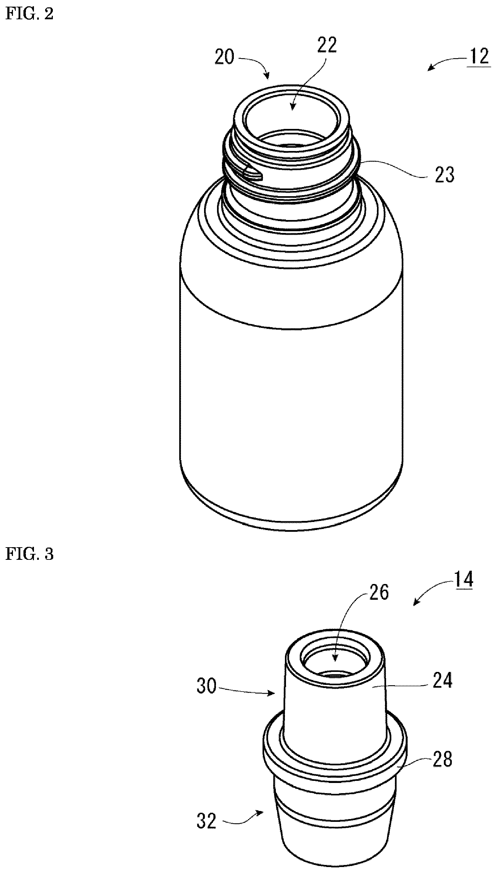

As shown in FIG. 2, the medical solution container body 12 is a closed-end tubular member having an approximately columnar shape. The medical solution container body 12 has an opening 20 provided with an inside stopper fittingly inserted portion 22 to which a lower end portion of the inside stopper 14 is fitted. Besides, the medical solution container body 12 is provided with a male threaded portion 23 on an upper end portion thereof such that the cap 18 is screwed thereonto.

As described above, the medical solution container body 12 according to the present exemplary embodiment has the approximately columnar shape; however, the shape of the medical solution container body 12 is not limited to this, and for instance, may be approximately prismatic or may be more complex. Besides, the material of the medical solution container body 12 is not particularly limited as well, and for instance, may be at least one selected from the group consisting of polypropylene (PP), polyethylene (PE), polyester (PET), and elastomer. However, polyethylene (PE) or elastomer is optimal when the medical solution applying container 10 is used in such a manner of dripping the medical solution from the brush 16, whereas polyethylene (PE) or polyester (PET) is preferred in attempt to make a user recognize the remaining amount of the medical solution.

As shown in FIGS. 3 to 5, the inside stopper 14 is a tubular member having an approximately columnar shape and includes an inside stopper body 24 and a brush inserted-and-mounted hole 26 provided in a lengthwise center part of the inside stopper body 24. Besides, the inside stopper body 24 is provided with an approximately circular flange 28 protruding from the outer periphery of an approximately middle part thereof. Moreover, the inside stopper body 24 is shaped such that an upper side of the flange 28 (a side to which the brush 16 is inserted and attached) has a relatively small outer diameter, whereas a lower side of the flange 28 (a side fitted to the medical solution container body 12) has a relatively large outer diameter. It should be noted that the inside stopper body 24 may be shaped such that the upper side of the flange 28 has a relatively large outer diameter, whereas the lower side of the flange 28 has a relatively small outer diameter.

In the inside stopper body 24, the upper side having the relatively small outer diameter is referred to as a brush attached portion 30, whereas the lower side having the relatively large outer diameter is referred to as a medical solution container body attached portion 32.

As described above, the inside stopper 14 according to the present exemplary embodiment has the approximately columnar shape; however, the shape of the inside stopper 14 is not limited to this, and for instance, may be approximately prismatic or may be more complex. Besides, the inside stopper 14 is configured to be fittingly inserted at the medical solution container body attached portion 32 into the inside stopper fittingly inserted portion 22 of the medical solution container body 12; instead, the inside stopper 14 may be configured to be attached to the medical solution container body 12 such that the lower end portion of the inside stopper 14 is put onto the upper end portion of the medical solution container body 12. Furthermore, the material of the inside stopper 14 is not particularly limited as well, and for instance, may be at least one selected from the group consisting of polypropylene (PP), polyethylene (PE), polyester (PET), and polybutylene terephthalate (PBT). What is required for the material of the inside stopper 14 is a stiffness that enables the brush 16 to be easily inserted into the brush inserted-and-mounted hole 26 and enables the medical solution container body attached portion 32 to be easily fittingly inserted into the inside stopper fittingly inserted portion 22.

The medical solution container body attached portion 32 includes an inside stopper internal space 34, having a greater diameter than the brush inserted-and-mounted hole 26, on the inner side thereof. The inside stopper internal space 34 is communicated with the lower end of the brush inserted-and-mounted hole 26. In the present exemplary embodiment, the brush inserted-and-mounted hole 26 has a circular cross section shaped to conform to the outer shape of the brush 16. However, as described below, the outer shape of the brush 16 is not limited to be columnar, and for instance, may be prismatic; hence, the shape of the brush inserted-and-mounted hole 26 can be changed to conform to the outer shape of the brush 16.

Besides, the brush inserted-and-mounted hole 26 is provided with a pressing-for-maintaining portion 36 protruding from the lower end portion thereof to the inside stopper internal space 34. In the present exemplary embodiment, the pressing-for-maintaining portion 36 is composed of four tongue pieces 38 disposed on an approximately common imaginary circle so as to enclose the lower end portion of the brush inserted-and-mounted hole 26. Moreover, the four tongue pieces 38 are disposed through gaps 40 formed therebetween. Each gap 40 extends along the brush inserted-and-mounted hole 26.

It should be noted that the number of the tongue pieces 38 disposed on the approximately common imaginary circle is not limited to four and can be assumed to be, for instance, two, three, six, eight, or so forth; however, it is preferred to set the number of the tongue pieces 38 to be four in terms of easiness in manufacturing the inside stopper 14, balance in holding the brush 16, and so forth.

The four tongue pieces 38 are downwardly tapered to approach the center of the brush inserted-and-mounted hole 26. The imaginary circle, formed by the lower ends (distal ends) of the tongue pieces 38, is set to be slightly smaller than the outer shape of the brush 16 to be inserted and mounted to the brush inserted-and-mounted hole 26. With this configuration, when the lower end of the brush 16 inserted into the brush inserted-and-mounted hole 26 reaches the pressing-for-maintaining portion 36, the tongue pieces 38 are configured to press together the lateral periphery of the lower end portion of the brush 16 so as to maintain the position of the brush 16 with respect to the inside stopper 14.

Furthermore, a plurality of vertical ribs 44 are provided to extend from positions, located slightly above roots 42 of the tongue pieces 38 on the inner surface of the brush inserted-and-mounted hole 26, to the inner surfaces of the tongue pieces 38, respectively.

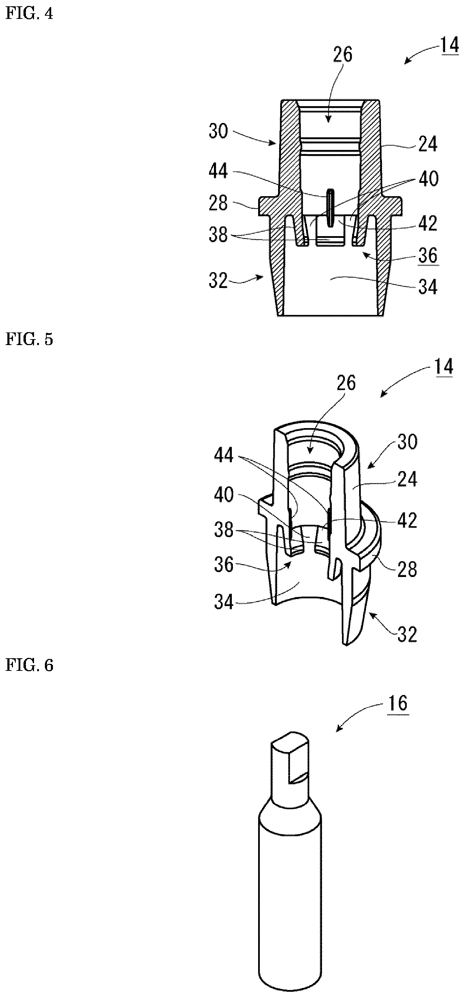

As shown in FIG. 6, the brush 16 is formed by collectively pressing a bundle of synthetic fibers having predetermined specific gravity and strength in an approximately columnar shape with a predetermined density. The medical solution L permeates the lower end portion of the brush 16 and is drawn to the upper end portion of the brush 16 by capillary action; hence, the medical solution L is configured to be applicable to a desired position on the skin or so forth by pressing the upper end of the brush 16 thereto.

As described above, the outer shape of the brush 16 according to the present exemplary embodiment has the approximately columnar shape; however, the outer shape of the brush 16 is not limited to this, and for instance, may be approximately prismatic or may be more complex. Besides, the material of the brush 16 is not particularly limited as well, and for instance, may be at least one selected from the group consisting of polyester (PET, PBT) fiber, polypropylene (PP) fiber, polyamide (PA) fiber, acrylic (PMMA) fiber, and polyacetal (POM) fiber.

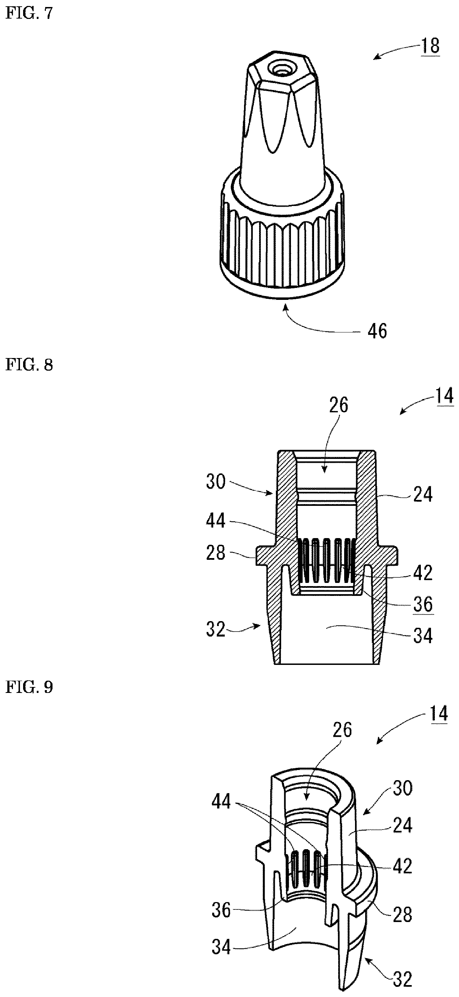

As shown in FIG. 7, the cap 18 is a member for avoiding the followings when the medical solution applying container 10 is stored without being used: unintended attachment of the medical solution L to an undesired object or so forth due to contact of the upper end of the brush 16 therewith; and unintended evaporation or so forth of the medical solution L through the upper end of the brush 16.

The cap 18 according to the present exemplary embodiment has an approximately conical shape and includes a brush accommodation space 46 on the inner side thereof to accommodate the distal end portion of the brush 16. Besides, the cap 18 is provided with a female threaded portion 47 on the lower end portion of the inner surface thereof to be screwed onto the male threaded portion 23 of the medical solution container body 12 (see FIG. 1).

As described above, the outer shape of the cap 18 according to the present exemplary embodiment has the approximately conical shape; however, the outer shape of the cap 18 is not limited to this, and for instance, may be approximately prismatic or columnar shape, or alternatively, may be more complex. Besides, the material of the cap 18 is not particularly limited as well, and for instance, may be at least one selected from the group consisting of polypropylene (PP), polyethylene (PE), and acrylonitrile butadiene styrene (ABS) as long as the material is not extremely inferior in moisture-proofing performance and in stiffness for preventing reduction in torque required for opening the cap 18.

### Assemblage of Medical Solution Applying Container 10

Referring back to FIG. 1, a procedure for assembling the medical solution applying container 10 according to the present exemplary embodiment will be briefly explained. The medical solution container body 12 is preliminarily filled up with a predetermined amount of medical solution L. Next, the medical solution container body attached portion 32 of the inside stopper 14 is fittingly inserted into the opening 20 of the medical solution container body 12. Insertion of the inside stopper 14 into the medical solution container body 12 continues until the flange 28 thereof is contacted to the end surface of the opening 20. Thereafter, the brush 16 is gradually inserted, from the lower end thereof, into the brush inserted-and-mounted hole 26 of the inside stopper 14. Insertion of the brush 16 into the inside stopper 14 continues to a position that the outer peripheral surface of the brush 16 is pressed and held by the respective tongue pieces 38 composing the pressing-for-maintaining portion 36. Finally, the cap 18 is set to cover the distal end portion of the brush 16. In this way, assemblage of the medical solution applying container 10 is completed.

The procedure for assembling the medical solution applying container 10 is not limited to the above. For example, the brush 16 may be preliminarily fitted into the inside stopper 14; thereafter, the medical solution container body attached portion 32 of the inside stopper 14 may be fittingly inserted into the opening 20 of the medical solution container body 12. Alternatively, the inside stopper 14 may be attached to the medical solution container body 12; thereafter, the medical solution container body 12 may be filled up with the medical solution L through the brush inserted-and-mounted hole 26 of the inside stopper 14.

### Features of Medical Solution Applying Container 10

In the inside stopper 14 for the medical solution applying container 10 according to the present exemplary embodiment, the brush inserted-and-mounted hole 26, to which the brush 16 is inserted and mounted, is provided at the lower end portion thereof with the pressing-for-maintaining portion 36 that presses the lateral periphery of the brush 16 so as to maintain the position of the brush 16 with respect to the inside stopper 14. Accordingly, depending on to what extent the lower end portion of the brush 16 is protruded and exposed from the brush inserted-and-mounted hole 26 to the interior of the medical solution container body 12 in which the medical solution L is stored, it is possible to adjust the amount of the medical solution L permeating the lower end portion of the brush 16.

Consequently, the extraction amount of the medical solution L can be herein easily adjusted, unlike in a conventional medical solution applying container, without necessity of adjusting the extraction amount of the medical solution L depending on the level of pinching or squeezing the medical solution container body 12.

Besides, the pressing-for-maintaining portion 36 in the inside stopper 14 is composed of the plural tongue pieces 38 disposed through the gaps 40 formed therebetween. Each gap 40 extends along the brush inserted-and-mounted hole 26. Accordingly, even when the medical solution L, stored in the medical solution container body 12, reduces in amount and is thereby disabled to permeate the brush 16 from the lower end surface thereof, the medical solution L is enabled to permeate the brush 16 from the outer peripheral surface of the lower end portion thereof through the gaps 40; hence, it is possible to provide the medical solution applying container 10 that the medical solution L, stored in the medical solution container body 12, can be easily used up without being left.

Furthermore, the vertical ribs 44 are provided to extend from the surface of the lower end portion of the brush inserted-and-mounted hole 26 to the inner surfaces of the tongue pieces 38, whereby each tongue piece 38 bends lesser at the root portion thereof (in the vicinity of the lower end portion of the brush inserted-and-mounted hole 26) but bends greater at the distal end portion thereof (the lower end portion of each tongue piece 38). With the configuration, even when the surface hardness is not uniform among products of the brush 16 because of difference in material and density among the products, a pressing-for-maintaining force acting on the brush 16 can be appropriately maintained.

### Modification 1

In the exemplary embodiment described above, the pressing-for-maintaining portion 36 of the inside stopper 14 is formed by disposing the plural tongue pieces 38 to be aligned on the approximately common imaginary circle. Instead of this, as shown in FIGS. 8 and 9, the pressing-for-maintaining portion 36 may have a continuous approximately cylindrical shape without being divided into the tongue pieces 38.

### Modification 2

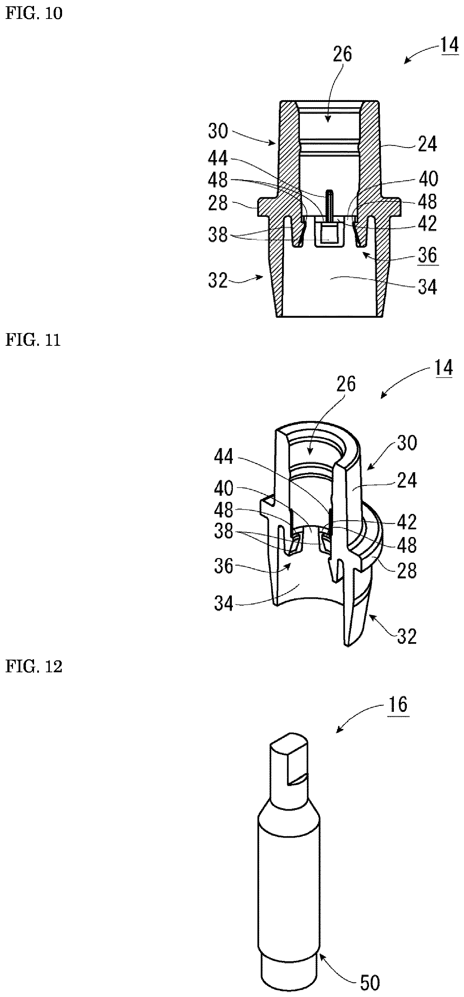

Alternatively, as shown in FIGS. 10 and 11, each tongue piece 38 may be provided with a hook portion 48, directed toward the upper end portion of the brush 16, in a contact position with the lateral periphery of the brush 16.

With the hook portions 48 formed as described above, it is made possible to produce a condition that the hook portions 48 lightly bite into the lateral periphery of the lower end portion of the brush 16. Therefore, the modification 2 is preferred in that when the medical solution applying container 10 is used by the user, occurrence of the following situation can be avoided: when the distal end portion of the brush 16 is pressed onto the skin or so forth, the brush 16 is deeply retracted into the inside stopper 14 without being intended by the user.

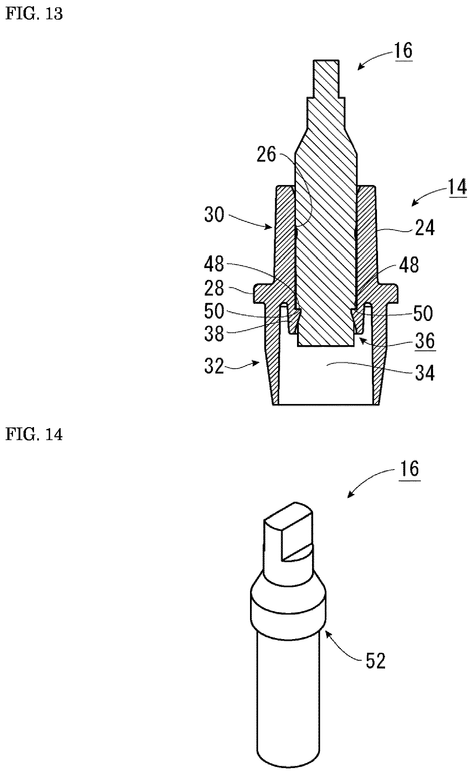

Furthermore, as shown in FIG. 12, the brush 16 is provided with a step 50 on the lateral periphery of the lower end portion thereof. The step 50 is hooked by the hook portions 48 provided in the inside stopper 14 as shown in FIG. 13. Therefore, the modification 2 is preferred in that it is made possible to precisely determine to what extent the brush 16 is fitted into the inside stopper 14; simultaneously, it is made possible to more reliably avoid the situation that the brush 16 is deeply retracted into the inside stopper 14 without being intended by the user.

### Modification 3

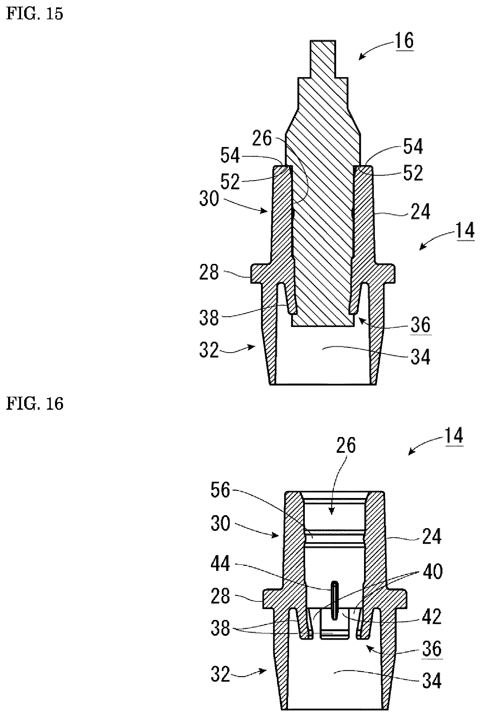

Alternatively, as shown in FIG. 14, the brush 16 is provided with a contact portion 52 protruding from an approximately middle portion of the lateral periphery thereof, whereas the inside stopper 14 is provided with a receiving portion 54, contacted by the contact portion 52, on the upper end of the brush inserted-and-mounted hole 26 (the upper end surface of the brush attached portion 30) as shown in FIG. 15. Therefore, the modification 3 is preferred in that it is made possible to precisely determine to what extent the brush 16 is fitted into the inside stopper 14; simultaneously, it is made possible to more reliably avoid the situation that the brush 16 is deeply retracted into the inside stopper 14 without being intended by the user.

### Modification 4

Alternatively, as shown in FIG. 16, the inside stopper 14 may be provided with an engaging portion 56 on a middle portion of the brush inserted-and-mounted hole 26 so as to be engaged with a middle portion of the lateral periphery of the brush 16.

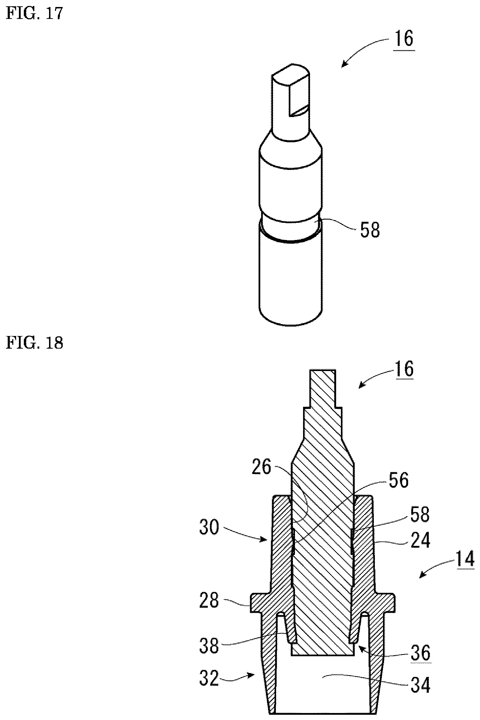

Specifically, as shown in FIGS. 17 and 18, it can be assumed that the inside stopper 14 is provided with a protruding portion inwardly protruding from the entire circumference of the middle portion of the brush inserted-and-mounted hole 26 as the engaging portion 56, whereas the brush 16 is provided with a recessed portion 58 on the entire circumference of an approximately middle portion thereof located in correspondence to the engaging portion 56 so as to be engaged therewith.

Contrarily, the inside stopper 14 may be provided with a recessed portion on the entire circumference of the middle portion of the brush inserted-and-mounted hole 26 as the engaging portion 56, whereas the brush 16 may be provided with a protruding portion protruding outward from the entire circumference of the approximately middle portion thereof located in correspondence to the engaging portion 56.

Consequently, the modification 4 is preferred in that it is made possible to precisely determine to what extent the brush 16 is fitted into the inside stopper 14; simultaneously, it is made possible to reliably avoid the situation that the brush 16 is deeply retracted into the inside stopper 14 without being intended by the user and a situation that the brush 16 is protruded from the inside stopper 14 to an extent not intended by the user.

### Modification 5

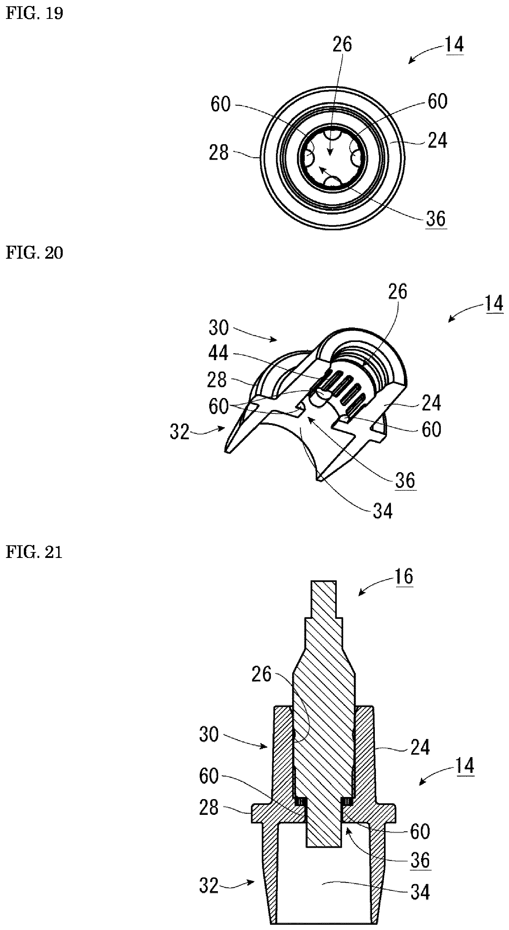

Alternatively, as shown in FIGS. 19 and 20, the pressing-for-maintaining portion 36 of the inside stopper 14 may be provided with a plurality of protruding portions 60 protruding from the inner surface of the brush inserted-and-mounted hole 26 toward the center of the brush inserted-and-mounted hole 26 so as to clog in part the brush inserted-and-mounted hole 26. In this case, as shown in FIG. 21, the lower end portion of the brush 16 is shaped to be fitted to gaps between the pressing-for-maintaining portion 36 (the protruding portions 60) and the brush inserted-and-mounted hole 26 without contacting the pressing-for-maintaining portion 36 (the protruding portions 60) clogging in part the brush inserted-and-mounted hole 26.

Consequently, the modification 5 is preferred in that it is made possible to prevent occurrence of a situation the brush 16 is rotated with respect to the inside stopper 14 without being intended by the user.

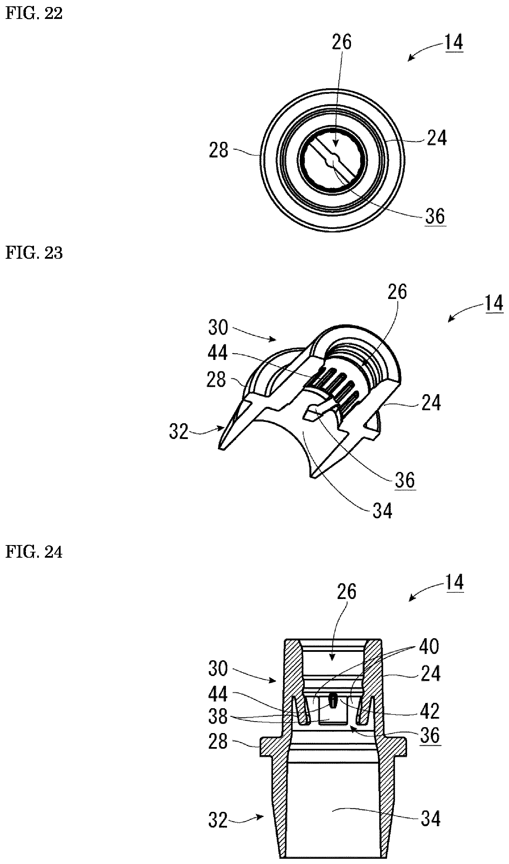

It should be noted that the following configuration may be provided as an alternative example of the modification 5. As shown in FIGS. 22 and 23, the pressing-for-maintaining portion 36 may be shaped to stretch from one side to another on the inner surface of the brush inserted-and-mounted hole 26 so as to traverse the cross-sectional middle of the brush inserted-and-mounted hole 26.

### Modification 6

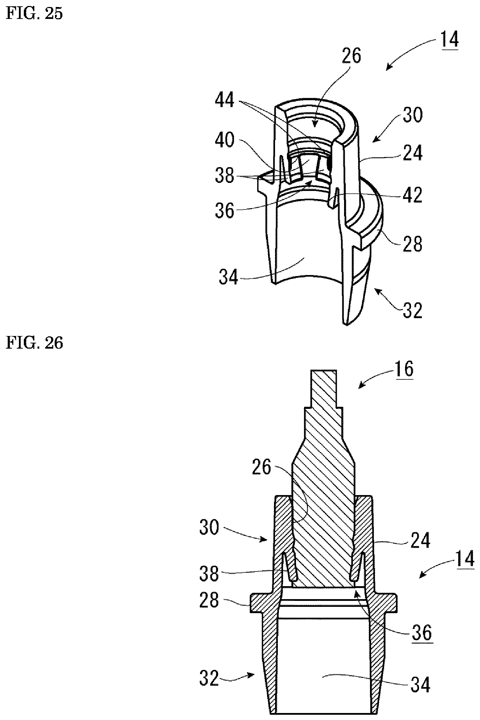

Alternatively, as shown in FIGS. 24 and 25, the tongue pieces 38, composing the pressing-for-maintaining portion 36, may be set to be located on the upper side in the inside stopper body 24; namely, the tongue pieces 38 may be shifted to shorten the dimension of the brush inserted-and-mounted hole 26 in the up-and-down direction. For example, in the modification 6, the pressing-for-maintaining portion 36 (the tongue pieces 38) is set to be located on the upper side of the flange 28.

Likewise in the inside stopper 14 according to the modification 6, as shown in FIG. 26, the brush 16 is gradually inserted, from the lower end thereof, into the brush inserted-and-mounted hole 26 of the inside stopper 14. Insertion of the brush 16 into the inside stopper 14 continues to a position that the outer peripheral surface of the brush 16 is pressed and held by the respective tongue pieces 38 composing the pressing-for-maintaining portion 36.

Accordingly, depending on to what extent the lower end portion of the brush 16 is protruded and exposed from the lower end of the brush inserted-and-mounted hole 26 (i.e., the lower ends of the tongue pieces 38) toward the medical solution container body 12, it is possible to adjust the amount of the medical solution L permeating the lower end portion of the brush 16.

### Modification 7

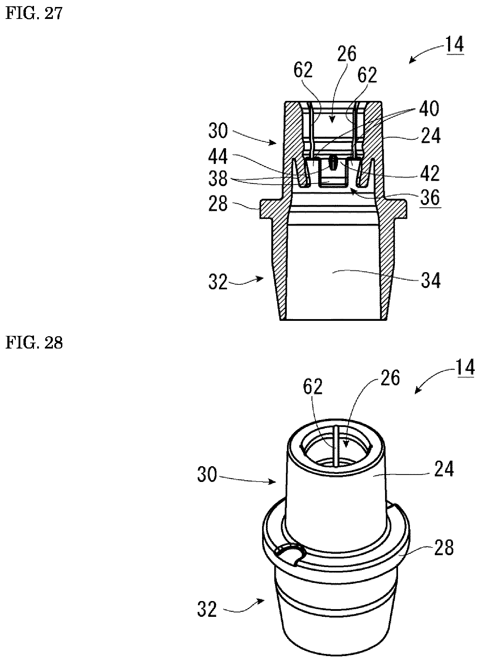

Alternatively, as shown in FIGS. 27 and 28, the inside stopper 14 may be provided with a plurality of recessed grooves 62 on the inner surface of the brush inserted-and-mounted hole 26. Each recessed groove 62 extends in a vertical direction from the opening end of the brush inserted-and-mounted hole 26 (the upper side in the drawings), into which the brush 16 is inserted and mounted, to the bottom end (the lower end) of the brush inserted-and-mounted hole 26 (the lower side in the drawings) on which the tongue pieces 38 (the pressing-for-maintaining portion 36) are provided. It should be noted that the number of the recessed grooves 62 may be single or plural. Moreover, each recessed groove 62 may be shaped straight as depicted in the drawings, or alternatively, may be shaped to be bent or curved.

When the medical solution applying container 10 is used after assembled, the medical solution L contained in the medical solution container body 12 is caused to permeate the brush 16 from the lower end portion thereof to the distal end thereof and is then applied to the skin or so forth. At this time, the recessed grooves 62 are provided to extend in the vertical direction from the opening end to the bottom end of the inner surface of the brush inserted-and-mounted hole 26; hence, the external air is likely to enter the medical solution container body 12 through the recessed grooves 62, whereby the medical solution L is enabled to permeate the brush 16 and reach the distal end thereof as much as possible.

It should be understood that the embodiment herein disclosed is illustrative only and is not restrictive in all aspects. It is intended that the scope of the present invention is indicated by the appended claims rather than the explanation described above and encompasses all the changes that come within the meaning of the appended claims.

### REFERENCE SIGNS LIST

- 10...: Medical solution applying container,
- 12...: Medical solution container body,
- 14...: Inside stopper,
- 16...: Brush,
- 18...: Cap,
- 20...: Opening,
- 22...: Inside stopper fittingly inserted portion,
- 23...: Male threaded portion,
- 24...: Inside stopper body,
- 26...: Brush inserted-and-mounted hole,
- 28...: Flange,
- 30...: Brush attached portion,
- 32...: Medical solution container body attached portion,
- 34...: Inside stopper internal space,
- 36...: Pressing-for-maintaining portion,
- 38...: Tongue piece,
- 40...: Gap,
- 42...: Root,
- 44...: Vertical rib,
- 46...: Brush accommodation space,
- 47...: Female threaded portion,
- 48...: Hook portion,
- 50...: Step,
- 52...: Contact portion,
- 54...: Receiving portion,
- 56...: Engaging portion,
- 58...: Recessed portion,
- 60...: Protruding portion,
- 62...: Recessed groove,
- L...: Medical solution

## Claims

1. A medical solution applying container (10) comprising:
a medical solution container body (12) for storing a medical solution (L);
an inside stopper (14) attached at a lower end portion thereof to the medical solution container body (12),
a brush (16) attached to an upper end portion of the inside stopper (14) so as to apply the medical solution (L),
the inside stopper (14) comprising:
a brush inserted-and-mounted hole (26) to which the brush (16) is inserted and mounted; and
a pressing-for-maintaining portion (36) provided in a lower end portion of the brush inserted-and-mounted hole (26), the pressing-for-maintaining portion (36) pressing a lateral periphery of the brush (16) so as to maintain a position of the brush (16) with respect to the inside stopper (14), wherein
the pressing-for-maintaining portion (36) includes a plurality of tongue pieces (38), and
the plurality of tongue pieces (38) are disposed through a plurality of gaps (40) formed therebetween, each of the plurality of gaps (40) extending along the brush inserted-and-mounted hole (26),
**characterized in that** it is possible to adjust the amount of the medical solution (L) permeating the lower end portion of the brush (16), depending on to what extent the lower end portion of the brush (16) is protruded and exposed from the brush inserted-and-mounted hole (26) towards the interior of the medical solution container body (12).

2. The medical solution applying container (10) according to claim 1, wherein a plurality of vertical ribs (44) are formed to extend from a surface of the lower end portion of the brush inserted-and-mounted hole (26) to inner surfaces of the plurality of tongue pieces (38).

3. The medical solution applying container (10) according to claim 1 or 2, wherein each of the plurality of tongue pieces (38) is provided with a hook portion (48) directed toward the upper end portion in a contact position with the lateral periphery of the brush (16).

4. The medical solution applying container (10) according to any one of claims 1 to 3, wherein the brush inserted-and-mounted hole (26) is provided with a receiving portion (54) on an upper end thereof, the receiving portion (54) contacted by a contact portion (52) protruding from a middle portion of the lateral periphery of the brush (16).

5. The medical solution applying container (10) according to any one of claims 1 to 4, wherein the brush inserted-and-mounted hole (26) is provided with an engaging portion (56) in a middle portion thereof, the engaging portion (56) engaged with the middle portion of the lateral periphery of the brush (16).

6. The medical solution applying container (10) according to any one of claims 1 to 5, wherein the brush inserted-and-mounted hole (26) is provided with a recessed groove (62) on an inner surface thereof, the recessed groove (62) extending from an opening end of the brush inserted-and-mounted hole (26) to a lower end thereof on which the pressing-for-maintaining portion (36) is provided.

## Patentansprüche

1. Behälter (10) zum Auftragen einer medizinischen Lösung, aufweisend:
einen Behälterkörper (12) zum Aufbewahren einer medizinischen Lösung (L);
einen inneren Stopfen (14), der an einem unteren Endabschnitt davon an dem Behälterkörper (12) für die medizinische Lösung angebracht ist,
eine Bürste (16), die an einem oberen Endabschnitt des inneren Stopfens (14) angebracht ist, um die medizinische Lösung (L) aufzutragen,
wobei der innere Stopfen (14) Folgendes aufweist:
ein in die Bürste eingesetztes und montiertes Loch (26), in das die Bürste (16) eingesetzt und montiert wird; und
einen Drücken-zum-Halten-Abschnitt (36), der in einem unteren Endabschnitt des in die Bürste eingesetzten und montierten Lochs (26) vorgesehen ist, wobei der Drücken-zum-Halten-Abschnitt (36) gegen einen seitlichen Umfang der Bürste (16) drückt, um eine Position der Bürste (16) in Bezug auf den inneren Stopfen (14) zu halten, wobei
der Drücken-zum-Halten-Abschnitt (36) mehrere Zungenstücke (38) aufweist, und
die mehreren Zungenstücke (38) durch mehrere dazwischen ausgebildete Spalte (40) angeordnet sind, wobei sich jeder der mehreren Spalte (40) entlang des in die Bürste eingesetzten und montierten Lochs (26) erstreckt,
**dadurch gekennzeichnet, dass** es möglich ist, die Menge der medizinischen Lösung (L), die den unteren Endabschnitt der Bürste (16) durchdringt, in Abhängigkeit davon einzustellen, in welchem Ausmaß der untere Endabschnitt der Bürste (16) von dem in die Bürste eingesetzten und montierten Loch (26) in Richtung des Inneren des Behälterkörpers (12) für die medizinische Lösung vorsteht und freiliegt.

2. Behälter (10) zum Auftragen einer medizinischen Lösung nach Anspruch 1, wobei mehrere vertikale Rippen (44) so ausgebildet sind, dass sie sich von einer Oberfläche des unteren Endabschnitts des in die Bürste eingesetzten und montierten Lochs (26) zu inneren Oberflächen der mehreren Zungenstücke (38) erstrecken.

3. Behälter (10) zum Auftragen einer medizinischen Lösung nach Anspruch 1 oder 2, wobei jedes der mehreren Zungenstücke (38) mit einem Hakenabschnitt (48) versehen ist, der in einer Kontaktposition mit dem seitlichen Umfang der Bürste (16) in Richtung des oberen Endabschnitts gerichtet ist.

4. Behälter (10) zum Auftragen einer medizinischen Lösung nach einem der Ansprüche 1 bis 3, wobei das in die Bürste eingesetzte und montierte Loch (26) an seinem oberen Ende mit einem Aufnahmeabschnitt (54) versehen ist, wobei der Aufnahmeabschnitt (54) von einem Kontaktabschnitt (52) kontaktiert wird, der von einem mittleren Abschnitt des seitlichen Umfangs der Bürste (16) vorsteht.

5. Behälter (10) zum Auftragen einer medizinischen Lösung nach einem der Ansprüche 1 bis 4, wobei das in die Bürste eingesetzte und montierte Loch (26) in seinem mittleren Abschnitt mit einem Eingriffsabschnitt (56) versehen ist, wobei der Eingriffsabschnitt (56) mit dem mittleren Abschnitt des seitlichen Umfangs der Bürste (16) in Eingriff steht.

6. Behälter (10) zum Auftragen einer medizinischen Lösung nach einem der Ansprüche 1 bis 5, wobei das in die Bürste eingesetzte und montierte Loch (26) an seiner inneren Oberfläche mit einer vertieften Nut (62) versehen ist, wobei sich die vertiefte Nut (62) von einem Öffnungsende des in die Bürste eingesetzten und montierten Lochs (26) zu seinem unteren Ende erstreckt, an dem der Drücken-zum-Halten-Abschnitt (36) vorgesehen ist.

## Revendications

1. Récipient d'application de solution médicale (10) comprenant :
un corps de récipient de solution médicale (12) pour stocker une solution médicale (L) ;
un bouchon intérieur (14) fixé au niveau d'une partie d'extrémité inférieure de celui-ci au corps de récipient de solution médicale (12),
une brosse (16) fixée à une partie d'extrémité supérieure du bouchon intérieur (14) de manière à appliquer la solution médicale (L),
le bouchon intérieur (14) comprenant :
un trou d'insertion et de montage de brosse (26) dans lequel la brosse (16) est insérée et montée ; et
une partie de pression pour le maintien (36) prévue dans une partie d'extrémité inférieure du trou d'insertion et de montage de brosse (26), la partie de pression pour le maintien (36) pressant une périphérie latérale de la brosse (16) de manière à maintenir une position de la brosse (16) par rapport au bouchon intérieur (14), dans lequel
la partie de pression pour le maintien (36) comprend une pluralité de pièces de languette (38), et
la pluralité de pièces de languette (38) sont disposées à travers une pluralité d'espaces (40) formés entre elles, chacun de la pluralité d'espaces (40) s'étendant le long du trou d'insertion et de montage de brosse (26),
**caractérisé en ce qu'**il est possible d'ajuster la quantité de la solution médicale (L) pénétrant dans la partie d'extrémité inférieure de la brosse (16), selon la mesure dans laquelle la partie d'extrémité inférieure de la brosse (16) fait saillie et est exposée à partir du trou d'insertion et de montage de brosse (26) vers l'intérieur du corps de récipient de solution médicale (12).

2. Récipient d'application de solution médicale (10) selon la revendication 1, dans lequel une pluralité de nervures verticales (44) sont formées pour s'étendre à partir d'une surface de la partie d'extrémité inférieure du trou d'insertion et de montage de brosse (26) jusqu'aux surfaces internes de la pluralité de pièces de languette (38).

3. Récipient d'application de solution médicale (10) selon la revendication 1 ou 2, dans lequel chacune de la pluralité de pièces de languette (38) est prévue avec une partie de crochet (48) dirigée vers la partie d'extrémité supérieure dans une position de contact avec la périphérie latérale de la brosse (16).

4. Récipient d'application de solution médicale (10) selon l'une quelconque des revendications 1 à 3, dans lequel le trou d'insertion et de montage de brosse (26) est prévu avec une partie de réception (54) sur une extrémité supérieure de celui-ci, la partie de réception (54) étant en contact avec une partie de contact (52) faisant saillie à partir d'une partie centrale de la périphérie latérale de la brosse (16).

5. Récipient d'application de solution médicale (10) selon l'une quelconque des revendications 1 à 4, dans lequel le trou d'insertion et de montage de brosse (26) est prévu avec une partie de mise en prise (56) dans une partie centrale de celui-ci, la partie de mise en prise (56) étant mise en prise avec la partie centrale de la périphérie latérale de la brosse (16).

6. Récipient d'application de solution médicale (10) selon l'une quelconque des revendications 1 à 5, dans lequel le trou d'insertion et de montage de brosse (26) est prévu avec une rainure évidée (62) sur une surface interne de celui-ci, la rainure évidée (62) s'étendant à partir d'une extrémité d'ouverture du trou d'insertion et de montage de brosse (26) jusqu'à une extrémité inférieure de celui-ci sur laquelle la partie de pression pour le maintien (36) est prévue.
